# EUROPEAN PATENT SPECIFICATION

(11) **EP 2 516 673 B1**
(45) Date of publication and mention of the grant of the patent: **23.09.2015**
(21) Application number: 10798090.6
(22) Date of filing: 21.12.2010
(51) Int. Cl.: C12Q 1/68, C12Q 1/70

(54) **PREDICTIVE VALUE OF IL28B GENE POLYMORPHISM COMBINED WITH PRETREATMENT SERUM IP-10 QUANTIFICATION FOR RESPONSE TO PEGINTERFERON AND RIBAVIRIN IS ENHANCED IN COMPARISON WITH ANY OF THESE BIOMARKERS ALONE**
DER PRÄDIKTIVE WERT DES IL28B-GEN-POLYMORPHISMUS IN KOMBINATION MIT DER IP-10-QUANTIFIZIERUNG IM SERUM VOR DER BEHANDLUNG FÜR DIE REAKTION AUF PEGINTERFERON UND RIBAVIRIN IST BESSER IM VERGLEICH ZU EINEM DIESER BIOMARKER ALLEINE
LA VALEUR PRÉDICTIVE DU POLYMORPHISME DU GÈNE IL28B COMBINÉE À LA QUANTIFICATION D'IP-10 DANS DU SÉRUM DE PRÉTRAITEMENT POUR LA RÉPONSE AU PÉGINTERFÉRON ET À LA RIBAVIRINE EST AMÉLIORÉE PAR RAPPORT À L'UN DE CES BIOMARQUEURS SEUL

(30) Priority: 22.12.2009 EP 09180387; 03.09.2010 EP 10175297
(43) Date of publication of application: 31.10.2012
(73) Proprietor: Janssen Sciences Ireland UC, Little Island, County Cork (IE)
(72) Inventor: AERSSENS, Jeroen, B-3221 Nieuwrode (Holsbeek) (BE); FANNING, Gregory Charles, B-1150 Woluwe St Pierre (Brussels) (BE); FRIED, Michael W., Chapel Hill North Carolina 27514 (US)
(74) Representative: van Wanrooij, Eva
(86) International application number: PCT/EP2010/070429
(87) International publication number: WO 2011/076814

(56) References cited:
- GE DONGLIANG ET AL: "Genetic variation in IL28B predicts hepatitis C treatment-induced viral clearance.", NATURE 17 SEP 2009 LNKD- PUBMED:19684573, vol. 461, no. 7262, 17 September 2009 (2009-09-17), pages 399-401, XP002602519, ISSN: 1476-4687
- TANAKA YASUHITO ET AL: "Genome-wide association of IL28B with response to pegylated interferon-alpha and ribavirin therapy for chronic hepatitis C.", NATURE GENETICS OCT 2009 LNKD- PUBMED:19749757, vol. 41, no. 10, October 2009 (2009-10), pages 1105-1109, XP002602520, ISSN: 1546-1718 cited in the application
- ROMERO ANA I ET AL: "Interferon (IFN)-gamma-inducible protein-10: association with histological results, viral kinetics, and outcome during treatment with pegylated IFN-alpha 2a and ribavirin for chronic hepatitis C virus infection", JOURNAL OF INFECTIOUS DISEASES, UNIVERSITY OF CHICAGO PRESS, CHICAGO, IL LNKD- DOI:10.1086/507307, vol. 194, no. 7, 1 October 2006 (2006-10-01), pages 895-903, XP009099562, ISSN: 0022-1899 cited in the application

### SEQUENCE LISTINGS

<110> Tibotec Pharmaceuticals
<120> Predictive value of IL28B Gene Polymorphism combined with Pretreatment Serum IP-10 Quantification for Response to Peginterferon and Ribavirin Is Enhanced in comparison with any of these Biomarkers alone
<130> TIP 218 PCT
<150> EP09180387.4
   <151> 2009-12-22
<150> EP10175297.0
   <151> 2010-09-03
<160> 3
<170> PatentIn version 3.3
<210> 1
   <211> 601
   <212> DNA
   <213> Homo sapiens
<300>
   <308> rs12979860
   <309> 2005-07-17
   <313> (301)..(301)
<400> 1
   caggactgcg gggacgagag ggcgttagag cgggccgcgc ccgggccatg cctctcccgc      60
   ccactcccgg gcctcaccga tggccgcgga ggatccctcc tggggcggaa ggagcagttg     120
   cgctgccccc agctcagcgc ctcttcctcc tgcgggacaa gcggcgctta tcgcatacgg     180
   ctaggccccc tcgccagggc ccctaacctc tgcacagtct gggattcctg gacgtggatg     240
   ggtactggca gcgcacggtc gtgcctgtcg tgtactgaac cagggagctc cccgaaggcg     300
   ygaaccaggg ttgaattgca ctccgcgctc ccccagcaaa gcccctcgcc ccgacctgga     360
   gccgagtcct cccggcaggg ctcccttctg tgattgaccc tgagcctgcg ttcgcgctga     420
   cgacggggac tgcgggggtc tcgtggtggg aattgtgggc gctgacatag gagaggcgcc     480
   tgctgggcgc taggacgcag gaccccttgg gacaggaacg ggtgtatggg aacccggtgg     540
   ggccagggtc ccagggggca caggggctgg gcggtgactt acgtagcggt ccctcagcgc     600
   c                                                                     601
<210> 2
   <211> 1001
   <212> DNA
   <213> Homo sapiens
<300>
   <308> rs12980275
   <309> 2004-03-20
   <313> (501)..(501)
<400> 2
   agagcaagac cctgtctcta aacttaaaga cagagagaga gagtccaggc gcggtagctc      60
   atgcctgtaa tcccagcact ttgggaggcc aaggccagtg gatcacctga gatcaggagt     120
   tcgagaccga cctggctaac atggtgaaac cccatttcta ctaaaactac aaaaaattag     180
   ctgggtgtgg tggcagacac ctgtaatccc agctactcgg gaggccgagg caggagattc     240
   acttgaaccc agcaggtgga ggttgcagtg agccaagatc gcgccactac actccagctt     300
   gggcaacaag agcgaaactc tgtctcaaaa aaaaaaaaaa aaaaaaagag agcaagagga     360
   gggaaggaag ttctgacaca tgctacaata aaaatgaact ttgaagacat tatgtcagtg     420
   aaataagcca gtctcaaaag aacaaatgct gtatgattcc ccctacatga ggtgctgaga     480
   gaagtcaaat tcctagaaac rgacgtgtct aaatatttgc cggggtagcg ggaggggtta     540
   ataggaagtt gttgtttaat gggtacagag attcaatttt gtaagatgaa gagagttctg     600
   gagattgctt gccgaacaat gtagcaaaca ccactaggac cagacctgta atcccggcac     660
   tttgggaggc caaggggggc agatcacttg aggtcaggag ttcgagacca acctggccaa     720
   catggcaaaa ccccatctct actaaaaata cagtaattag ccaggcatct gactggctaa     780
   tggcacacac ctgtagtctc agctactcag gaggctgagg tgggaggatc acttgagcct     840
   aggaggtcga ggctgcaatg agctatgatc atgccagtgc cctccagcca gggtgacaga     900
   gcaaggtcct gtctcaaaaa aaaaaaaata gaataaataa ataataaaat tgcactactg     960
   aacaatgtgc tttagaaagg ataagatggt acattttgtg g                        1001
<210> 3
   <211> 601
   <212> DNA
   <213> Homo sapiens
<300>
   <308> rs8099917
   <309> 2005-07-18
   <313> (301)..(301)
<400> 3
   tgaaaaatac acaaaaacat aaaagaaaaa aaacatcacc tataacttca ccatcctcct    60
   ctcatccctc atcccacttc tggaacaaat cgtcccaata cataggaatt ttccatgtgt   120
   ttatttgtgc atatgttttc tgactaccaa agtaacactt gttccttgta aaagattcca   180
   tccatacaaa aacatacaac atggagagtt aaagtaagtc ttgtatttca cctcctggag   240
   gtaaatattt tttaacaatt tgtcactgtt cctccttttg ttttcctttc tgtgagcaat   300
   ktcacccaaa ttggaaccat gctgtataca gtttggtagc tggcttttta tgtcttacca   360
   ttatctctca tttgcattct cccacatctt taattatagc gtatcagtta gggcccagca   420
   ggaaacagat ggcccgtcaa tttaggataa tttgaggtgg ggttgatacc agaagccttt   480
   ataaagcgat tggatagtaa aggcaaatga caagagctag tgcaagctcc tggggccagc   540
   atgggtgagg ggcctcatcc acaggcctaa aggaagggga gagggttgag ggtgtgcatg   600
   t                                                                   601

## Description

Hepatitis C virus (HCV), a member of the *flaviviridae* family, is a blood-borne virus that causes chronic inflammation of the liver, and can lead to liver fibrosis, cirrhosis and carcinoma. World-wide, approximately 170 million people are infected with HCV and treatment options are limited. Current standard of care for the treatment of chronic hepatitis C consists of a weekly injection of pegylated interferon alpha (IFN-α) and twice daily oral administration of ribavirin (Fried and Hadziyannis, 2004, Semin Liver Dis 24 Suppl 2, 47-54). However, only 40-50% of patients infected with genotypes 1 or 4 treated with a 48 week regimen achieve a sustained virologic response (SVR) compared to 80% of the patients infected with genotypes 2 or 3. Clearly, therefore a viral genotype component to IFN-α / ribavirin treatment response exists. Moreover, current standard of care is often associated with adverse effects such as depression and anemia (Fried and Hadziyannis, 2004). Therefore, there is an unmet medical need for novel, more efficacious and safer treatment options for HCV patients.

So the standard of care for HCV genotype 1 treatment currently includes 48 weeks of pegylated IFN-α and ribavirin, with a follow-up time of 24 weeks to determine if sustained virological response has been achieved. A sustained virological response (SVR) is defined as the absence of detectable HCV RNA for at least 24 weeks after stopping therapy. The duration, cost and morbidity related to treatment with these agents has established a desire in the field to better understand response and to beforehand identify patients that will benefit from a modification of the treatment strategy.

Recently data has been generated indicating a genetic contribution to IFN-α response, with a strong link between *IL28B* polymorphisms and clinically determined clearance of virus in HCV-infected individuals from various ethnic backgrounds. This data supports a host component to treatment failure previously implicated by cytokine serum markers, the most consistent being that of IP-10 serum levels measured prior to the commencement of treatment.

IFN-λ proteins, which have demonstrated anti-HCV activity, are encoded by the *IL28A*/*B* and *IL29* genes (Kotenko, et al 2003, Nature Immunol. 4:69; Sheppard et al 2003, Nature Immunol. 4:63); the *IL28B* gene encoding interferon-λ3 (IFN-λ3). Recent studies have identified a single nucleotide polymorphism (SNP) (rs12979860, SEQ ID NO: 1) three kilobases (3kb) upstream of the *IL28B* gene as a potential predictive marker of treatment response (Ge et al 2009, Nature 461:399). This finding was confirmed by other independent studies using other SNP markers (rs12980275, SEQ ID NO: 2 and rs8099917, SEQ ID NO: 3) located closely to the IL28B gene (Suppiah et al 2009, Nature Genetics 41:1100; Tanaka et al 2009, Nature Genetics 41:1105). The advantageous variants were shown to associate strongly with more than a two-fold difference in response to HCV drug treatment. Across a multi-ethnic population, approximately 80% of patients who carry two copies of the advantageous variant cleared the virus during IFN-α therapy and remained virus-free for a period of 24 weeks posttreatment. The genetic association appears to be important in response prediction in all ethnic groups studied including European, African, and Asian patients. These studies also provide preliminary evidence that the mRNA expression of *IL28B* in peripheral blood mononuclear cells (PBMC) is affected by the *IL28B* genotype linked with increased response to IFN-α treatment. In a later study this genotype was also shown to strongly enhance the natural resolution and treatment induced viral clearance of HCV infection among individuals of both European and African ancestry (Thomas et al 2009, Nature 461:798, Ge Dongling et al 2009, 461:399). These studies have provided some promise that response to IFN-α could be predicted in a personalized medicine setting.

Prior to the identification of IFN-γ-inducible protein 10 (IP-10 or CXCL10) as a marker for non-response, tumor necrosis factor α (TNF-α), interleukin (IL) 1 B, IL-10, and IL-8 had been identified as serum markers of non-response to IFN-α treatment. IP-10 was shown to be elevated in HCV patients both within the liver and in serum, and it was found that these levels were more elevated in non-responder HCV patients than in responders to IFN-α. This observation was then confirmed in larger datasets including the European Caucasian DITTO-study where baseline levels were the most important determinant for treatment response prediction (Romero et al 2006, JID 194:895; Lagging et al 2006, Hepatology 44:1617). Subsequently, the IP-10 findings were extended to HCV/HIV co-infected patients (Zeremski et al 2007, JAIDS 45:262) which was confirmed subsequently in a second study and additionally correlated with hepatic inflammation and fibrosis in these patients (Reiberger et al 2008, Antivir Ther 13:969). The IP-10 association has also been shown in different racial groups. (Fried et al, AASLD 2008, poster 1223)

A generalized view has emerged in the field that a heightened immune response indicated at the RNA, cellular or protein level may indicate less response to IFN-α / ribavirin therapy, although this has not reached a level of analysis or sophistication to be used clinically. Despite the known markers, the mechanism related to non-response remains unclear. Moreover, while promising, the *IL28B* genotype does not explain all aspects of interferon response and how this finding relates to other known biomarkers, such as viral genotype, viral load, weight, fibrosis, and IP-10 is not yet clear. The molecular link between the activity of the IL28B gene product IFN-λ3 and expression of immune genes suggests that IL28B and downstream cytokine and chemokine levels should be dependent and therefore downstream serum markers such as IP-10 would be effectively describing the same phenotypic phenomena.

The recently described genetic polymorphism in *IL28B* is extremely exciting and can yield important prognostic information for achieving sustained virological response to therapy with peginterferon and ribavirin. However, its clinical utility as a standalone test remains limited. For CC genotypes, the likelihood of SVR is estimated to be approximately 80%. In contrast for the TC heterozygote, the likelihood of response decreases to approximately 50%. Thus, the negative or positive predictive value of the genotyping test is relatively low, which will diminish its use in decision making for antiviral therapy. Methods to build upon its predictive capabilities utilizing the genetic test in combination with other readily available markers of antiviral response may improve the positive and negative predictive values and improve its clinical utility.

Disclosed is an analysis of HCV samples in Caucasians and African Americans wherein it has been unexpectedly determined that IP-10 serum levels and *IL28B* genotype are complementary and independent predictors of response, and specifically that IP-10 levels measured in HCV infected patients heterozygous for the *IL28B* genotype or homozygous for the non-responder genotype provide additional value in determining response to IFN-α treatment.

The *IL28B* polymorphic marker rs12979860 was tested using the ABI TaqMan allelic discrimination kit on 80 participants from VIRAHEP-C, an NIH-funded, clinical trial. Genotyped patients were part of a sub-study for whom a panel of serum cytokines was measured in archived sera (Fried et al, EASL 2008). The present cohort included 40 SVR and 40 non-responders, of which 39 were Caucasian and 41 African-American. The CC, CT, or TT genotypes were found in 19%, 61%, and 20%, respectively, with corresponding SVR rates of 87%, 47%, and 25% (Chi²=13.4, p=0.0012), which is in line with the initial report of Ge et al (2009, Nature 461:399).

Mean serum IP-10 levels were similar and appeared not to be associated with *IL28B* genotype (p=0.28). An SVR rate of 78% was found in patients with low (<600 pg/ml) versus 36% in patients with high (>600 pg/ml) IP-10 levels (Chi²=17.1, p<0.0001).

Serum IP-10 level within the *IL28B* genotype groups provided additional and independent information regarding the likelihood of SVR (Chi²=27.5, p<0.0001). More specifically, CT carriers with low IP-10 had 79% SVR versus 34% with high IP-10 levels. Among TT carriers a similar relationship was identified (50% for low IP-10 versus 10% for high IP-10).

| **IL28B genotype** | **CC** | **CT** | **TT** |
|---|---|---|---|
| **% SVR for entire cohort** (n) | **87%** (13/15) | **47%** (23/49) | **25%** (4/16) |
| **% SVR for IP-10 < 600 pg/ml** (n) | **100%** (7/7) | **79%** (11/14) | **50%** (3/6) |
| **% SVR for IP-10 > 600 pg/ml** (n) | **75%** (6/8) | **34%** (12/35) | **10%** (1/10) |

So, when *IL28B* genotype is combined with pretreatment serum IP-10 measurement in non-CC genotypes, the predictive value for individual patients is improved by further discriminating between SVR and non-response to peginterferon and ribavirin. More specifically, generalized linear model fitting of SVR including both *IL28B* genotype and serum IP-10 as the explanatory variables demonstrates a significant contributive effect of *IL28B* genotype (p<0.0013) and serum IP-10 (p<0.0016) but not of the interaction between *IL28B* and IP-10 (p=0.75) in estimating sustained virological response.

Hence, the current invention in general relates to a method of combining *IL28B* genotype determination with pre-treatment serum level measurement of IP-10 to predict the outcome of sustained virological response (SVR) or non-response to peginterferon and ribavirin for individual patients infected with HCV.

In the broader sense the invention relates to a method of combining two or more parameters selected from the group consisting of the pre-treatment serum level measurement of IP-10, the *IL28B* genotype determination, race or Hepatitis C viral load determination to predict the outcome of sustained virological response (SVR) or non-response to peginterferon and ribavirin for individual patients infected with HCV.

In the method wherein the *IL28B* genotype determination with pre-treatment serum level measurement of IP-10 is combined, either one or both of the parameters race or Hepatitis C viral load determination can be added for the prediction of the outcome of sustained virological response (SVR) or non-response to peginterferon and ribavirin for individual patients infected with HCV.

Onecharacteristic of the invention is that the *IL28B* genotype comprises the polymorphic marker rs12979860.

Unexpectedly, combining the results for *IL28B genotype* and serum IP-10 improves the negative predictive value among TT or TC genotypes for sustained virological response.

Part of the invention is also a diagnostic assay comprising means for at least the determination or measurement of *IL28B* polymorphism and IP-10 levels in serum from an HCV-infected patient.

### Example section

### EXAMPLE 1

### Patients and Treatment

The VIRAHEP-C study was a multicenter study of combination peginterferon and ribavirin therapy of chronic hepatitis C designed to assess the rates and predictors of response among AA and CA with genotype 1 infection and to identify reasons for nonresponse to therapy. The design and primary outcomes of the VIRAHEP-C trial have been reported elsewhere *[*Conjeevaram, H.S., et al., Peginterferon and ribavirin treatment in African American and Caucasian American patients with hepatitis C genotype 1. Gastroenterology, 2006. 139(2): p. 470-7*].* Patients 18 years and older who were treatment-naive, infected with genotype 1, had detectable HCV RNA, and had histologic evidence of chronic HCV were eligible to participate. Patients with a history of alcohol consumption of more than 2 drinks or the equivalent (20 g) per day or evidence of alcohol abuse in the preceding 6 months were excluded. All patients had undergone liver biopsy within 18 months of enrollment. Patients were classified by race as either African American or Caucasian race and by ethnicity as either Hispanic or non-Hispanic based on self-report. All participants were required to have been born in the United States.

Patients received peginterferon alfa-2a (Pegasys, Roche Pharmaceuticals, Nutley, NJ) 180µg weekly and ribavirin (Copegus, Roche Pharmaceuticals, Nutley,NJ) 1000-1200 mg daily for at least 24 weeks. Patients who became HCV RNA negative by week 24 continued treatment for a total of 48 weeks, whereas those who remained HCV RNA positive stopped treatment and were considered non-responders (NR). The primary endpoint of the trial was a sustained virological response (SVR), defined as the absence of detectable HCV RNA for at least 24 weeks after stopping therapy.

### Analysis of Serum Cytokines including IP-10

Fifty sustained virologic responders (SVR) and 50 non-responders (NR) to antiviral therapy were chosen from the VIRAHEP-C cohort and included in the cytokine analyses. No relapsers to 48 weeks of IFN based therapy were included in this retrospective analysis. The cohort consisted of 51 African Americans (AA) and 49 Caucasian Americans (CA). The cohort included 41 females and 59 males.

Fifty different cytokines were measured in the serum samples collected at baseline using the commercially available human 23-plex and 27-plex panel cytokine BioPlex assays (BioRad, catalog #171-A11123 and #171-A11127, respectively) on a Luminex system, according to the instructions of the manufacturer. Out of the 50 cytokines analyzed in the multiplex assays at baseline, 37 could be reliably quantified in the majority of the samples. Special attention was given to the analysis of serum IP-10 levels, that were determined as part of the human cytokine 27-plex panel BioPlex assay. The dynamic range of the IP-10 measurement in this assay is 3.1 - 50,938 pg/ml, with a limit of detection at 6.5 µg/ml.

### IL28B Genotyping

Genotyping was performed on a subset of 80 patients (40 responders, 40 non-responders) of whom serum IP-10 concentrations were analyzed (see above). This patient subset comprised 39 AA and 41 CA.

The *IL28B* polymorphic marker rs12979860 was tested using the ABI TaqMan allelic discrimination kit and the AB17900HT Sequence Detection System (Applied Biosystems), as described by Thomas et al (Nature, 461:798-802, 2009*).*

### Statistical analysis

Statistical analysis was done using SAS or JMP software (SAS Institute Inc). Graphs were made with Tibco Spotfire software or GraphPad Prism 4 software.

### IP10 is associated with SVR

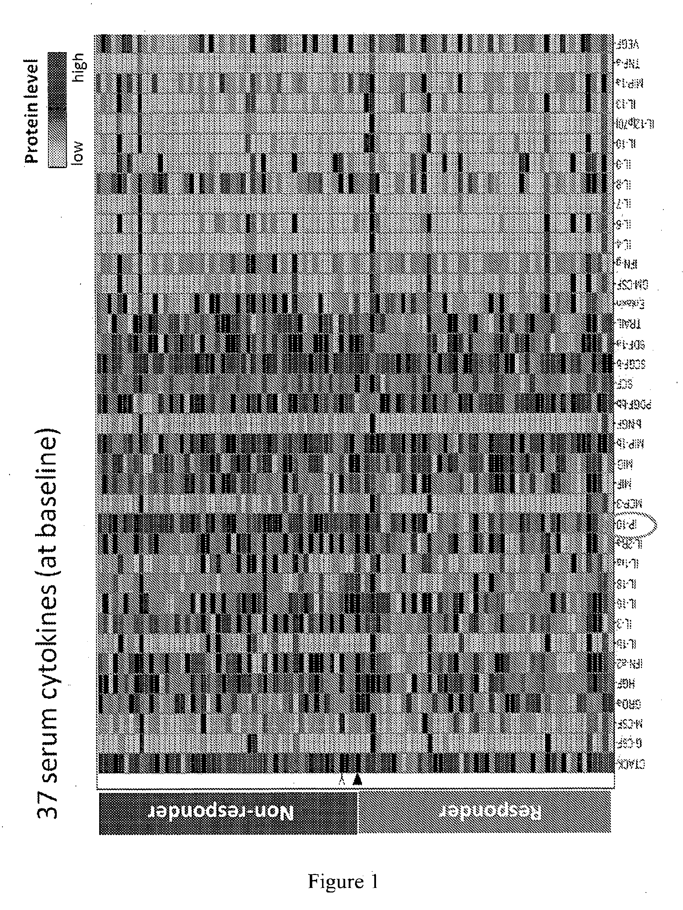

Serum samples from 100 patients in the VIRAHEP-C cohort undergoing HCV treatment have been evaluated using a comprehensive array of 50 cytokines and chemokines in order to determine if pre-treatment profiles of selected cytokines were predictive of treatment response (sustained virological response, SVR) in a well-characterized American cohort. Out of the 50 cytokines analyzed, 37 could be reliably measured, as depicted in the heat map analysis of responders versus non-responders in **FIGURE 1**. Baseline levels of all cytokines, except for IP-10 (see below), were not significantly different between responders and non-responders.

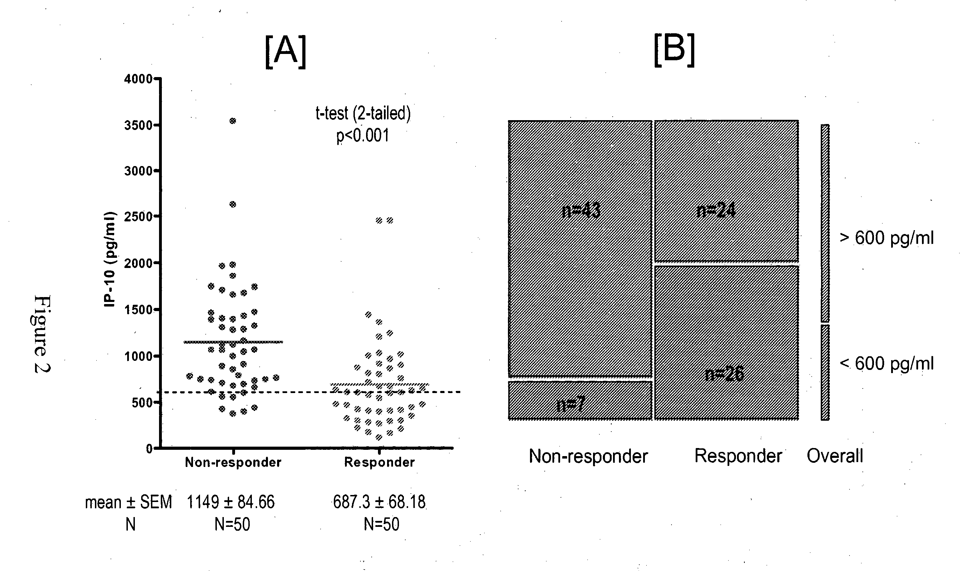

Mean serum IP-10 concentration at baseline was significantly lower in responders compared to nonresponders (687 ± 68 pg/ml versus 1149 ± 85 pg/ml, p<0.001) (**FIGURE 2A**). To assess the potential predictive value of IP-10 measurements patients were stratified according to a 600 pg/ml threshold value, similar as was applied in other studies earlier.

*[*Zeremski, M., et al., Interferon gamma-inducible protein 10: a predictive marker of successful treatment response in hepatitis C virus/HIV-coinfected patients. J Acquir Immune Defic Syndr, 2007. 45(3): p. 262-8 // Butera, D., et al., Plasma chemokine levels correlate with the outcome of antiviral therapy in patients with hepatitis C. Blood, 2005. 106(4): p. 1175-82*.* // Diago, M., et al., Association of pretreatment serum interferon gamma inducible protein 10 levels with sustained virological response to peginterferon plus ribavirin therapy in genotype 1 infected patients with chronic hepatitis C. Gut, 2006. 55(3): p. 374-9*.* // Lagging, M., et al., IP-10 predicts viral response and therapeutic outcome in difficult-to-treat patients with HCV genotype 1 infection. Hepatology, 2006. 44(6): p. 1617-25*.* // Narumi, S., et al., Expression of IFN-inducible protein-10 in chronic hepatitis. J Immunol, 1997. 158(11): p. 5536-44*.* // Romero, A.I., et al., Interferon (IFN)-gamma-inducible protein-10: association with histological results, viral kinetics, and outcome during treatment with pegylated IFN-alpha 2a and ribavirin for chronic hepatitis C virus infection. J Infect Dis, 2006. 194(7): p. 895-903*.]*

Seventy-nine percent (26/33) of individuals with a low baseline IP-10 level (<600 pg/ml) will be responders (positive predictive value = 79%), while 64% (43/67) of the individuals with a high baseline IP-10 level (>600 pg/ml) will be nonresponders to therapy (negative predictive value = 64%) (**FIGURE 2B**). Overall, this results in a specificity of 52% (26/50) and a sensitivity of 86% (43/50) for a test predictive of therapy response based on pre-treatment serum IP-10 levels.

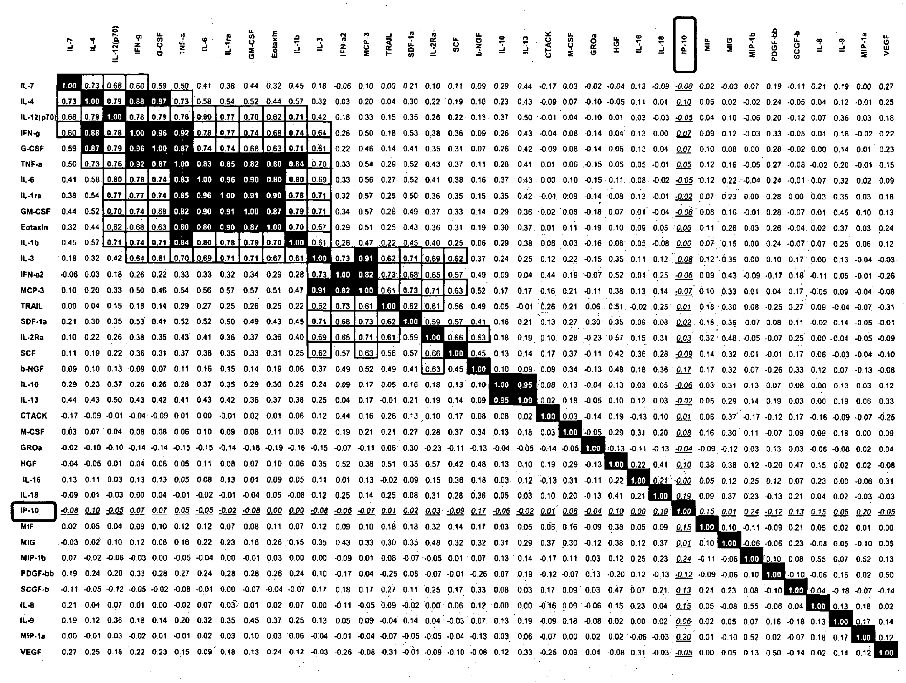

Based on a correlation matrix analysis using the 37 cytokines evaluated, no other cytokine correlated with IP-10 at baseline (**FIGURE 3**). Several groups of cytokines were noted to correlate with each other at baseline but none were associated with treatment response. Importantly, analysis of the 37 cytokines using a cross validation model revealed that no cytokine added to baseline IP-10 improved its misclassification rate of responders versus nonresponders (approximately 30%).

### IL28B genotype is associated with SVR

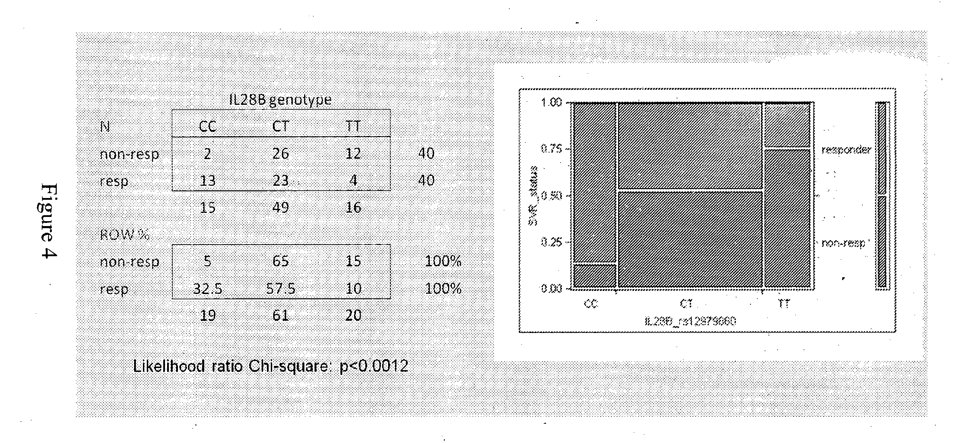

Stratification of the patient population according to *IL28B* genotype revealed a significant (likelihood ratio chi-square: p<0.0012) association with treatment response (**FIGURE 4**), with the CC genotype being associated with high SVR rate (87% SVR in CC genotype), whereas the TT genotype was associated with non-responders (75% non-responders in TT genotype).

### Additive and independent effect of IP10 serum level and IL28B genotype

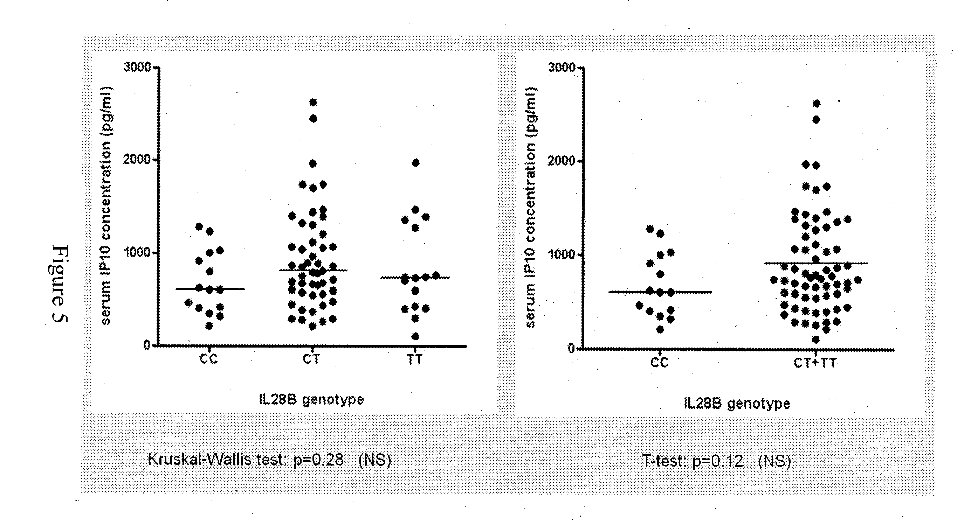

Mean serum IP-10 levels were not significantly different between the three *IL28B* genotype groups, also not when the CC genotype was compared versus the combined CT and TT genotype groups (**FIGURE 5**).

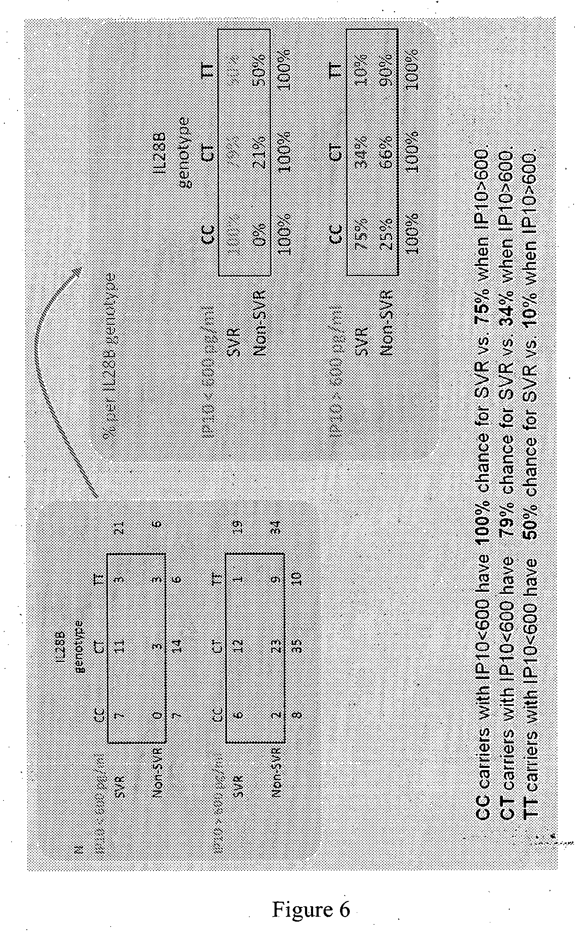

Next, patients were stratified according to IP-10 level above or below the 600 pg/ml threshold value that has been used in several studies before (see above). The stratification based on pretreatment serum IP-10 level was then analyzed in combination with IL28B genotype. The tabulated data of this combined analysis are provided in **FIGURE 6**.

This finding demonstrates that the predictivity for individual patients of (non-) response to peginterferon and ribavirin treatment can be significantly improved by a combined analysis of IL28B genotype and pretreatment serum IP-10 concentration.

More specifically, CT carriers with low IP-10 had 79% SVR versus 34% with high IP-10 levels. Among TT carriers a similar relationship was identified (SVR=50% for low IP-10 versus 10% for high IP-10). (See **Table 1**)

**TABLE 1 Summary table of the results of the combined analysis of IL28B genotype and serum IP-10 concentration (above or below 600 pg/ml) in relation to therapy outcome.**

| IL28B genotype | CC | CT | TT |
|---|---|---|---|
| % SVR for entire cohort (n) | 87% (13/15) | 47% (23/49) | 25% (4/16) |
| % SVR for IP-10 < 600 pg/ml (n) | 100% (7/7) | 79% (11/14) | 50% (3/6) |
| % SVR for IP-10 > 600 pg/ml (n) | 75% (6/8) | 34% (12/35) | 10% (1/10) |

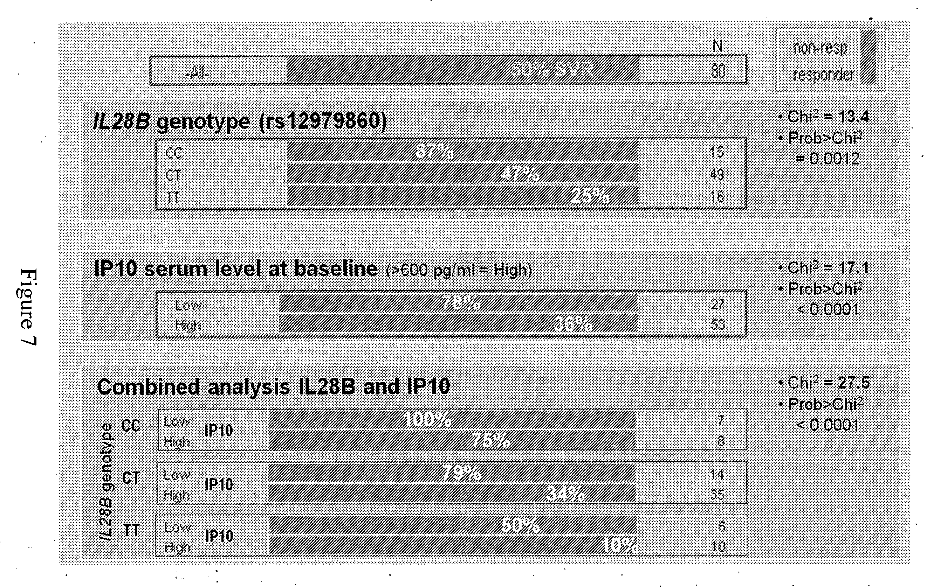

**FIGURE 7** provides a comparative overview of the individual analyses (IL28B genotype and IP-10 level) and the combined analysis. The chi-square test for homogeneity that was performed examines whether two or more populations (e.g. different IL28B genotype groups) have the same proportion of observations with a common characteristic (i.e. SVR response). Both of the individual analyses are significantly predictive for treatment response. Yet there is a clear additive effect when combining both markers, resulting in improved predictive value for individual patients, both of treatment response and non-response. This combined analysis results in a highly significant stratification of responders versus non-responders. More specifically, generalized linear modeling (SAS proc genmod) of SVR including both IL28B genotype and serum IP-10 (above or below 600 pg/ml) as the explanatory variables demonstrates a significant contributive effect of IL28B genotype (p<0.0013) and serum IP-10 (p<0.0016) but not of the interaction between IL28B and IP-10 (p=0.75) in estimating sustained virological response.

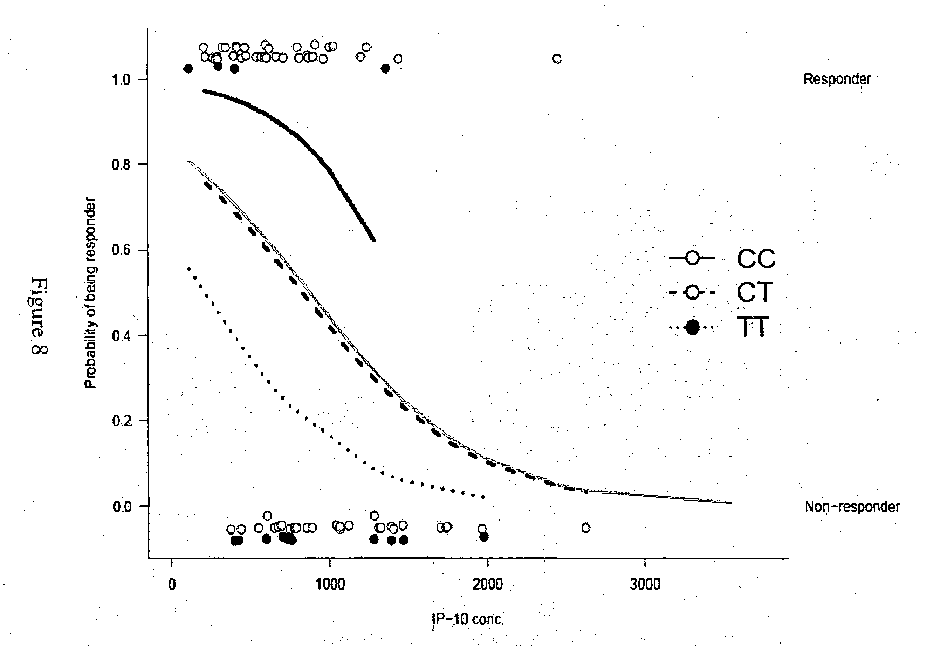

Finally, logistic regression modeling of SVR response based on serum IP-10 levels (treated as a continuous variable) and *IL28B* genotype (categorical variable) clearly separate responders and non-responders according to serum IP-10 level, with an IL28B genotype dependent shift in response curve (FIGURE 8).

Together, these findings demonstrate that the predictive value of a combination of pretreatment serum IP-10 level and IL28B genotype outperforms each of the individual markers in predicting sustained virological response to peginterferon and ribavirin therapy, and holds the promise to guide personalized medicine in HCV patients.

### EXAMPLE 2

In this study pre-treatment IP-10 levels was measured in serum samples from 272 patients in the VIRAHEP-C cohort (115 non-responders and 157 SVR). This analysis demonstrated IP-10 to be equally predictive of SVR in both CA and AA patients. The combination of pretreatment serum IP-10 levels with *IL28B* genotype was then assessed as predictors of response to pegIFN and ribavirin in this cohort.

### PATIENTS AND METHODS

**Patients.** The VIRAHEP-C study was a multicenter study of combination pegIFN and ribavirin therapy of chronic hepatitis C designed to assess the rates and predictors of response among AA and CA with genotype 1 infection, and to identify reasons for nonresponse to therapy. The design and primary outcomes of the VIRAHEP-C trial have been reported elsewhere *[*Conjeevaram, H.S., et al., Peginterferon and ribavirin treatment in African American and Caucasian American patients with hepatitis C genotype 1. Gastroenterology, 2006. 131(2): p. 470-7*].* Adults who were treatment naive, infected with genotype 1, had detectable HCV RNA, and had histologic evidence of chronic HCV were eligible to participate. Patients were classified by race as either African American or Caucasian, and by ethnicity as either Hispanic or non-Hispanic, based on self-report. All participants .were required to have been born in the United States. From 8 clinical centers across the United States, 401 patients were enrolled and started on therapy between July 2002 and December 2003.

For the present study, serum samples were acquired from a subset of 272 patients from the total VIRAHEP-C cohort, comprising 157 sustained virological responders (SVR) (104 CA, 53 AA) and 115 non-responders (34 CA, 81 AA). All specimens analyzed in this study were obtained under IRB-approved protocols for which participants provided written informed consent, including consent for genetic testing.

**Treatment.** Patients received peginterferon alfa-2a (Pegasys, Roche Pharmaceuticals, Nutley, NJ) 180ug weekly and ribavirin (Copegus, Roche Pharmaceuticals, Nutley, NJ) 1000-1200 mg daily for at least 24 weeks. Patients who became HCV RNA negative by week 24 continued treatment for a total of 48 weeks, whereas those who remained HCV RNA positive stopped treatment and were considered non-responders. The primary endpoint of the trial was SVR, defined as the absence of detectable HCV RNA for at least 24 weeks after stopping therapy.

**HCV RNA quantification.** HCV RNA testing was done at a central laboratory (SeraCare BioServices, Gaithersburg, MD) using the Cobas Amplicor Assay (sensitivity 50 IU/ml: Roche Molecular Diagnostics, Alameda, CA). Selected samples were tested for HCV RNA levels by Cobas Amplicor Monitor Assay and for HCV RNA genotype by Versant HCV Genotype Assay (Bayer, Tarrytown, NY).

**Liver histology.** All patients had undergone liver biopsy within 18 months of screening, which were read blinded by a central pathologist. All biopsies were assessed for severity of hepatitis C by grading the inflammation and staging the fibrosis using Ishak's modified histologic activity index (HAI) scoring system. **Quantification of serum IP-10.** Interferon-γ Inducible Protein-10 (IP-10) was measured in serum samples collected at baseline, prior to initiation of treatment, using the commercially available Quantikine human CXCL10/IP-10 immunoassay (R&D Systems). All samples were diluted 1:2 and analyzed in duplicate. The linear dynamic range of the IP-10 measurement in this assay was 8 - 500 pg/ml, with a detection limit at 7.8 pg/ml. Samples with IP-10 concentration above 1000 pg/ml were diluted 1:5 and re-analyzed.

**Genotyping of *IL28B.*** The *IL28B* polymorphic marker rs12979860 was analyzed using the ABI TaqMan allelic discrimination kit and the ABI7900HT Sequence Detection System (Applied Biosystems), as described by Thomas et al (Nature, 461:798-802, 2009*).* DNA samples were genotyped from 210 patients from our cohort.

**Statistical methods.** Standard statistical analyses were done using JMP 7.0.2 or SAS version 9.1 software (both from SAS Institute, Inc). IP-10 concentrations were log-transformed before use in statistical tests to meet distribution normality assumptions. Publicly available packages in R (version 2.8.0) were used to assess different classification models (diagonal linear discriminant analysis, random forest, support vector machine, and bagging), as well as receiver operating characteristic (ROC) curve analysis. Fitting logistic regression models and generalized linear models was performed using respectively the *proc logistic* and *proc genmod* procedures in SAS. Graphs were made with the utilized statistical software tools or with GraphPad Prism 4 (GraphPad Software, Inc).

### RESULTS

### Patients included in the study cohort.

Serum samples from 157 sustained virologic responders (SVR) and 115 non-responders to antiviral therapy were included from the VIRAHEP-C cohort for this study. The definitions of SVR and non-responder are provided in the above section. Patients with viral relapse, breakthrough or less than 12 weeks of available virologic data were excluded. The cohort consisted of 134 AA and 138 CA. Baseline patient characteristics of this cohort were as follows: age 48.4 ± 7.4 years; viral load 4.6 ± 5.7 X10⁶ IU/ml; platelet count 214 ± 73 X10⁶ cells/mm³; alanine transaminase (ALT) 90.9 ± 72.9 IU/I; total bilirubin 0.70 ± 0.35 mg/dl; albumin 4.1 ± 0.40 g/dl; and hematocrit 43.2 ± 3.8 % (all data as mean ± SD). The cohort included 96 females and 176 males, and 19% with an Ishak fibrosis score of 4-6. Samples from 210 of the 272 patients in our cohort were available for *IL28B* genotyping, 123 SVR and 87 non-responders of whom 111 were CA and 99 AA.

### Baseline serum IP-10 measurement and treatment response.

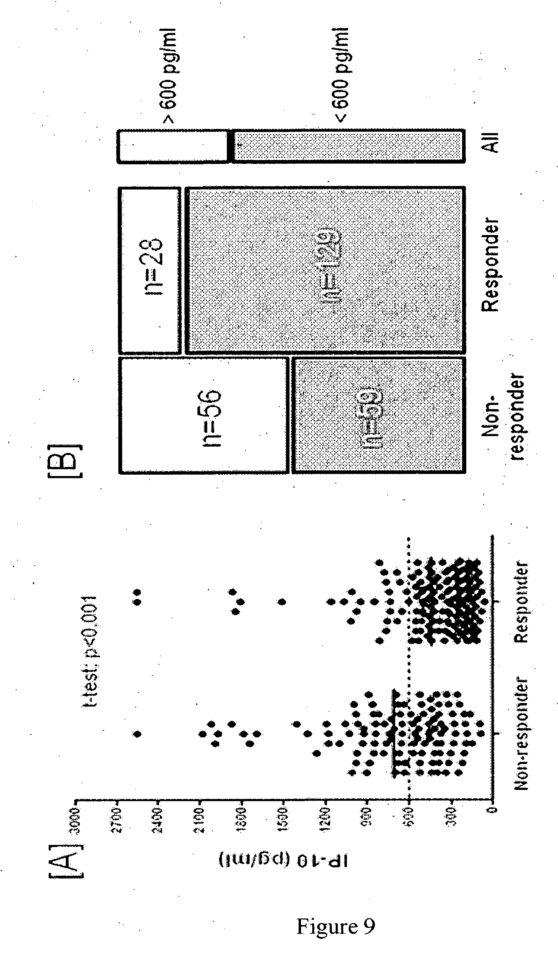

Mean serum IP-10 levels were significantly lower in SVR compared to non-responder patients (437 ± 31 pg/ml versus 704 ± 44 pg/ml, p<0.001) (**Figure 9A****, Table 2**). To assess the potential predictive value of IP-10 measurements, we stratified the patients according to a 600 pg/ml threshold value which has been used in prior studies. Sixty-nine percent (129/188) of individuals with a low baseline IP-10 level (<600 pg/ml) were responders (positive predictive value, PPV = 69%), while 67% (56/83) of the individuals with a high baseline IP-10 level (>600 pg/ml) were non-responders to therapy (negative predictive value, NPV = 67%) (**Figure 9B**). Overall, this result in specificity of 82% (129/157) and a sensitivity of 49% (56/115) for a test predictive of therapy response based on pre-treatment serum IP-10 levels.

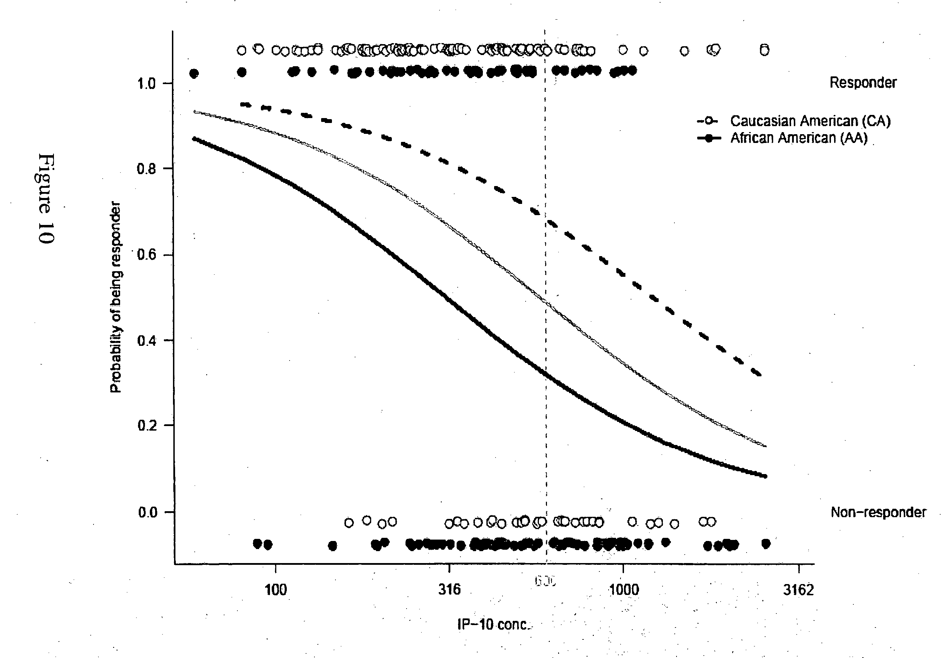

We evaluated the IP-10 threshold value of 600 pg/ml used to identify SVR patients by computing the cutoff value with the best discriminatory ability based on a ROC curve analysis. In our dataset, a threshold concentration of 370 pg/ml revealed the optimal combination of specificity (80%) and sensitivity (56%) in predicting SVR patients. We then determined our optimal IP-10 level to correctly predict both SVR as well as non-response. A threshold value of 550 pg/ml yielded the highest rate of true positives or negatives (69%), and correlated well with the 600 pg/ml cutoff that has been used in the literature (68% true positives or negatives predicted in our dataset). Finally, logistic regression analysis of pre-treatment IP-10 concentrations enabled fitting the probability of SVR for specific IP-10 levels measured in individual patients, and demonstrated a highly significant effect of IP-10 (p<0.0001; **Figure 10**, grey curve).

### Effect of race on serum IP-10 levels.

When comparing pre-treatment IP-10 serum levels of CA and AA, no significant differences were observed in separate analyses of the responders (p=0.75) and non-responders (p=0.97) (**Table 2**). The significant (p=0.015) difference in baseline serum IP-10 level between CA and AA that was observed in the overall study cohort can most likely be explained by the unbalanced composition of the cohort (IFN treatment response rate in the CA subgroup was 75% as compared to 40% in the AA subgroup). The highly significant difference in IP-10 serum level between responders and non-responders to IFN therapy was found in both CA and AA (Table 2). Logistic regression analyses of baseline IP-10 levels were used to generate treatment response curves for CA and AA patients (**Figure 10**). The response curves for AA and CA patients revealed a significant effect of both IP-10 (p<0.0001) and race (p<0.0001), but no significant interaction between IP-10 and race (p=0.08).

### IL28B genotype and treatment response.

Of the 210 patients genotyped, 30% were CC, 49% were CT, and 21% were TT. A significant association between *IL28B* genotype and treatment response was observed: corresponding SVR rates were 87% for CC, 50% for CT, and 39% for TT (p<0.0001) (**Table 3**). For Caucasian-Americans, 49% were CC with an SVR of 91%, 41% were CT with an SVR of 67% and 10% were TT with an SVR of 45% (p<0.001). For African-Americans, only 9% were CC with an SVR of 67%, 58% were CT with an SVR of 35% and 33% were TT with an SVR of 36% (p=0.20).

### Association of IL28B genotype and pretreatment IP-10 levels with treatment response.

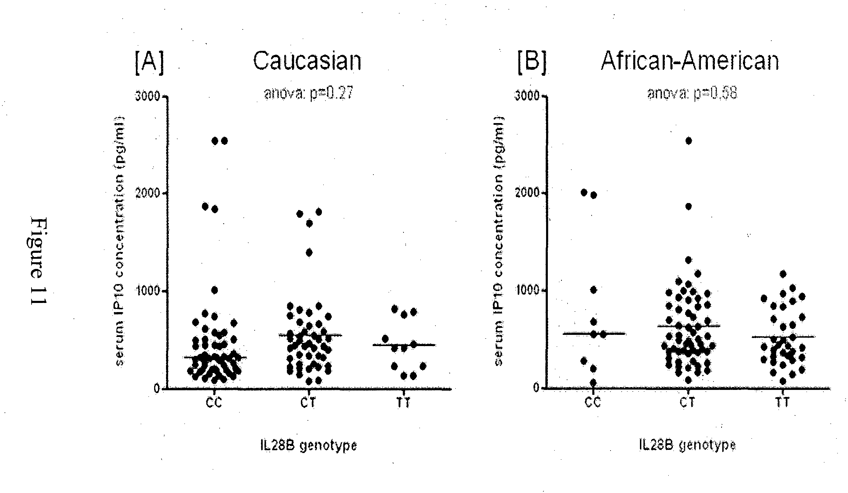

Mean serum IP-10 levels were similar for all patients regardless of *IL28B* genotype both in Caucasians (p=0.27) and in African-Americans (p=0.58) (**Figure 11**). This lack of correlation between serum IP-10 and *IL28B* genotype indicates that the associations with SVR observed for both of these markers are independent. Using the 600 pg/ml cutoff for pretreatment IP-10 levels, the SVR rate for our cohort of patients with both serum IP-10 and *IL28B* genotype data available (n=210) was 69% for those with a low IP-10 level (<600 pg/ml) and 35% for those with a high IP-10 level (>600pg/ml) (p<0.0001).

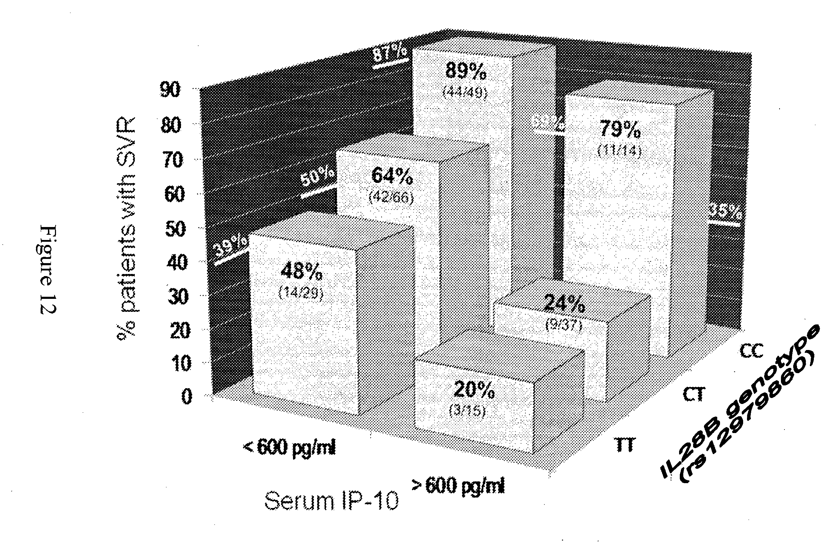

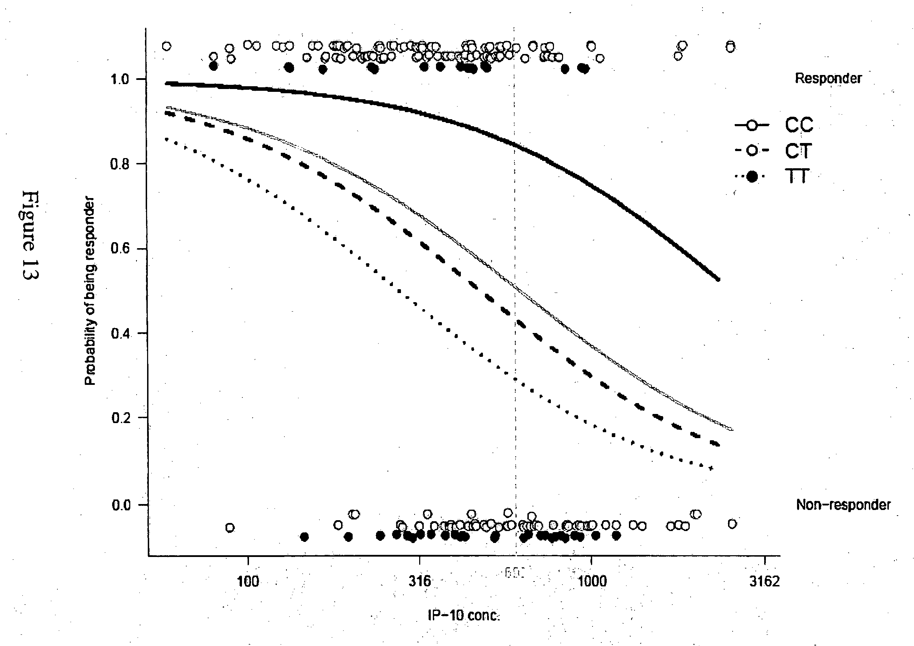

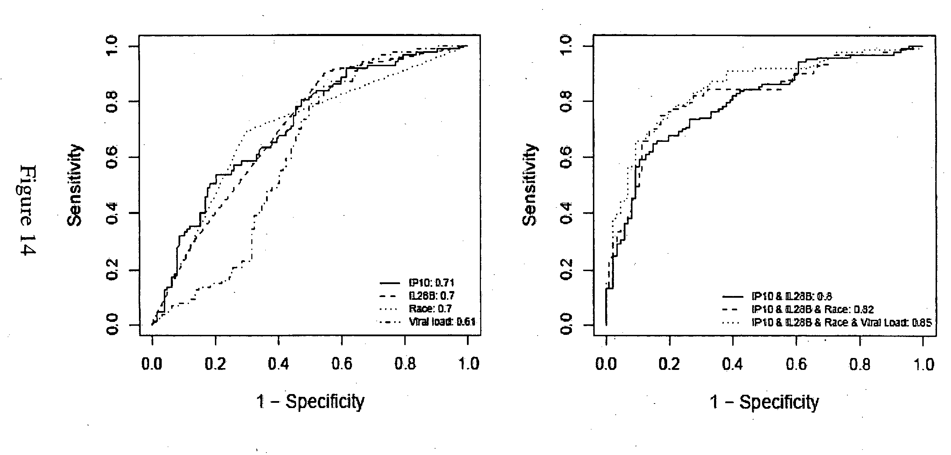

Modeling SVR as a function of *IL28B* genotype and serum IP-10 (above or below 600 pg/ml) in a nominal logistic regression revealed a significant additive effect of *IL28B* genotype (p<0.0001) and serum IP-10 (p<0.0015) in predicting SVR (Chi²=55, p<0.001), but no interaction between *IL28B* and IP-10 (p=0.66). **Figure 12** visualizes that baseline IP-10 levels within the *IL28B* genotype groups provided additional and independent information regarding SVR rate. More specifically, baseline IP-10 levels were most helpful in *IL28B* T-allele carriers. The overall response rate for CT carriers was 50% but for those with low IP-10 levels 64% had an SVR versus 24% with high IP-10 levels. For the TT genotype, 39% had an SVR with 48% in the low pretreatment IP-10 group and 20% in the high IP-10 group. Logistic regression modeling of SVR response based on serum IP-10 level treated as a continuous variable and *IL28B* genotype enabled a more individualized prediction of the probability of SVR according to serum IP-10 level, with an additional and significant *IL28B* genotype-dependent shift in response curve (Figure 13). Complementary receiver operating characteristic (ROC) curve analyses, which allow a more quantitative comparison of predictive models, revealed similar ROC area under the curve (AUC) values for the model based on pretreatment serum IP-10 alone (0.71) versus *IL28B* genotype alone (0.70). A much higher ROC AUC value (0.80) was achieved, however, for the model that combined both markers (**Figure 14**). Together, these data demonstrate that combining *IL28B* genotype with pretreatment serum IP-10 measurements clearly improves the predictive value of SVR, especially in non-CC genotypes.

The same and significant trend was also found when the analysis was done per racial group (**Table 4**). For example, in African Americans, the difference with baseline IP-10 levels was even more striking for the CT and TT *IL28B* genotypes. For the CT carriers with low IP-10, SVR was 48% versus 17% with high IP-10, whereas for TT carriers with low IP-10, SVR was 43% versus 25% with high IP-10.

### Combining IL28B genotype and pretreatment IP-10 levels with other baseline parameters to predict treatment response.

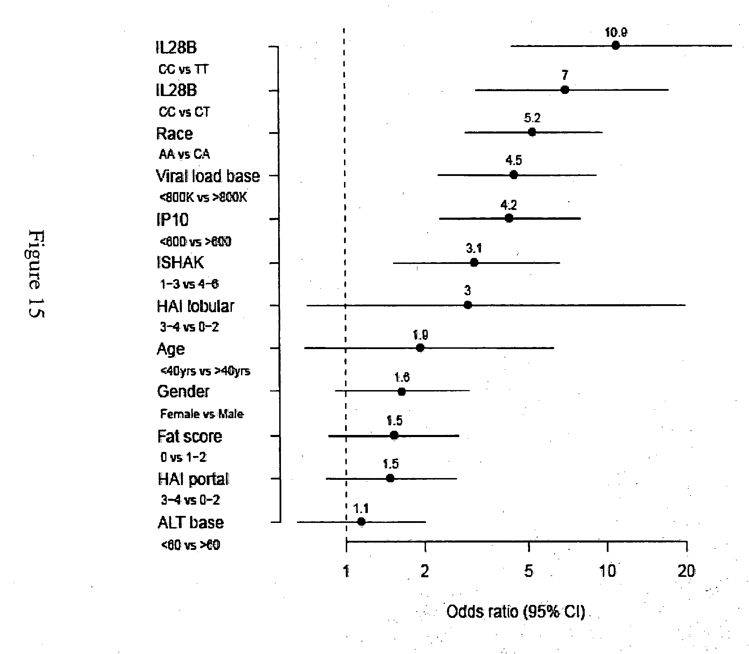

We assessed whether other baseline parameters, in addition to *IL28B* genotype and serum IP-10, could significantly improve the prediction of SVR. In this analysis, we added age, gender, race, pretreatment viral load, Ishak fibrosis score, ALT, steatosis, and histological activity index in a logistic regression model. Of all parameters included, only pre-treatment viral load (p<0.0001), *IL28B* genotype (p=0.0004), baseline IP-10 level (p=0.0033), and race (p=0.0011) significantly contributed to the model. No interaction between any pair of variables was significant (all p>0.1). When all variables were treated as categorical variables (eg, IP-10 above or below 600 pg/ml, rather than as a continuous variable), the resulting generalized linear model included the same four significant variables plus ISHAK fibrosis score (**Figure 15**).

ROC curve analyses were performed to compare the predictive power of models that include the most significant variables identified in these multivariate analyses (**Figure 14**). The AUC of the model that combined *IL28B* genotype and serum IP-10 (AUC 0.80) clearly outperformed the models based on the individual variables, including the model based on *IL28B* genotype alone (AUC 0.70). The addition of race and baseline viral load further improved the model, although the added gain was modest (AUC up to 0.85).

**TABLE 2. Pretreatment serum IP-10 levels (pg/ml) in HCV patients stratified according to race and interferon treatment response (SVR). A significant difference was found between CA and AA for the whole cohort (p=0.015), but not within the subgroups of responders or non-responders.**

| | All | SVR | Non-responder | t-test |
|---|---|---|---|---|
| All | | 437 ± 31 (n=157) | 704 ± 44 (n=115) | p<0.001 |
| | | | | |
| Caucasian | 504 ± 38 (n=138) | 447 ± 44 (n=104) | 677 ± 69 (n=34) | p<0.001 |
| African-American | 598 ± 38 (n=134) | 418 ± 35 (n=53) | 716 ± 55 (n=81) | p<0.001 |

**TABLE 3. SVR rate according to IL28B genotype in the entire cohort and stratified per racial group.**

| IL28B genotype | CC | CT | TT | Likelihood Ratio Chi² |
|---|---|---|---|---|
| % SVR entire cohort (n SVR / total n) | 87% (55/63) | 50% (51/103) | 39% (17/44) | p < 0.0001 |
| % SVR CA (n SVR CA / total n CA) | 91% (49/54) | 67% (31/46) | 45% (5/11) | p = 0.0008 |
| % SVR AA (n SVR AA / total n AA) | 67% (6/9) | 35% (20/57) | 36% (12/33) | p = 0.20 |

**TABLE 4. SVR rate (%) according to a combination of IL28B genotype and pretreatment serum IP-10 level (<600 pg/ml: low; >600 pg/ml: high), stratified per racial group. Data are expressed as % SVR (n SVR / total n). Likelihood ratio Chi² analyses were performed to test for response homogeneity across the sample groups.**

| | Caucasian-American | | African-American | |
|---|---|---|---|---|
| IP-10 | low | high | low | high |
| *IL28B* | | | | |
| CC | 91% (40/44) | 90% (9/10) | 80% (4/5) | 50% (2/4) |
| CT | 79% (26/33) | 38% (5/13) | 48% (16/33) | 17% (4/24) |
| TT | 62% (5/8) | 0% (0/3) | 43% (9/21) | 25% (3/12) |
| | Chi² = 25.5, p<0.0001 | | Chi² = 11.8, p=0.037 | |

### Description of Figures 1-15

**FIGURE 1****.** *Heatmap representing pretreatment serum concentrations of 37 cytokines measured in 50 responding (SVR) and 50 nonresponding HCV patients on Interferon α* / *ribavirin therapy.*
**FIGURE 2****.** *IP-10 measurements at baseline in responders versus non-responders to IFN therapy. **[A]** A significantly (p<0.001) lower IP-10 serum level was observed at baseline in patients responding to IFN treatment versus non-responders. Horizontal lines represent mean values per group. To assess the potential predictive value of IP-10 measurements individuals were stratified according to a 600 pglml threshold value (dashed line). **[B]** A significantly smaller proportion of subjects with high (>600 pglml) IP-10 levels was found in the responder as compared to the non-responder group (Pearson Chi square = 16.3, p<0.001).*
**FIGURE 3****.** *Correlation matrix including 37 cytokines measured at baseline in 100 HCV patients (pretreatment samples). Blue boxes indicate correlations between cytokines above 60% (light blue), 70% (middle blue), or 80% (dark blue), respectively. The red boxes indicate the correlations of IP-10 with all other 36 cytokines (all below 60%).*
**FIGURE 4****.** *IL28B genotype (rs12979860) distribution in responders and nonresponders to IFNα+ribavirin treatment. Data are shown as number of patients (n) or as percentage of responders or nonresponders, respectively (Row%). A graphical representation is shown at the right side of the figure.*
**FIGURE 5****.** *IL28B genotype (rs12979860) in relation to pre-treatment serum IP-10 concentrations in 80 HCV patients. Patients were either grouped per IL28B genotype (left), or by comparing the CC genotype versus CT*/*TT genotypes (right). No significant IL28B genotype associated differences were observed.*
**FIGURE 6****.** *Combined analysis of IL28B genotype and serum IP-10 concentration (above or below 600 pg*/*ml) in relation to therapy outcome. The figure shows the number of patients (N) (left panel)* as *well as the fraction (as* %) *of patients per genotype and IP-10 level that responded or not to IFNα*/*ribavirin treatment.*
**FIGURE 7****.** *Graphical summary comparing the predictive value of treatment outcome in HCV patients, based on IL28B genotype only (upper panel), IP-10 serum level only (middle panel), and a combined analysis of IL28B genotype and serum IP-10 level. The percentages in the bars represent the fraction* of *SVR responders in the particular subgroup. The total number of patients in each subgroup (bar) are shown at the right side* of *the bars.*
**FIGURE 8****.** *Graphical representation of a logistic regression analysis fitting sustained virological response (SVR) to peginterferon and ribavirin treatment based on serum IP-10 levels (grey curve). Next, IL28B genotype was added into the model, resulting in a regression curve for each of the genotypes (CC: blue, CT: red, TT: green). The relatively small number of patients in each of the subgroups might explain that only the contribution of serum IP-10 was revealed as a significant (p=0.017) contributor in this model but not IL28B genotype (p=0.31) or the interaction between IP-10 and IL28B (p=0.93). Each patient sample is represented as a circle at the upper (responder) or lower (non-responder) part of the figure; the colors of the circles represent the IL28B genotype.*
**FIGURE 9****.** Pretreatment serum IP-10 (pg/ml) in responder (SVR) and non-responder patients. (A) Distribution graph of IP-10 data; (B) Mosaic plot based on IP-10 levels (stratified above or below 600 pg/ml) and treatment response outcome.
**FIGURE 10****.** Graphical summary of a logistic regression analysis fitting the probability of being a responder (SVR) based on pretreatment serum IP-10 levels (pg/ml), in the overall cohort (gray curve) or stratified per racial group (Caucasian-American: red, African-American: blue). Each patient sample is represented as a circle at the upper (SVR) or lower (non-responder) part of the figure. The logistic regression model revealed a significant effect of IP-10 (p<0.0001) and of race (p<0.0001), but no significant interaction between IP-10 and race (p=0.08).
**FIGURE 11****.** Pretreatment serum IP-10 (pg/ml) in HCV patients stratified according to *IL28B* genotype in (A) Caucasian-Americans and (B) African-Americans. One-way ANOVA demonstrated no significant differences in IP-10 level according to *IL28B* genotype.
**FIGURE 12****.** Sustained virological response (SVR) rate (%) in HCV patients stratified according to pretreatment serum IP-10 (above versus below 600 pg/ml) and *IL28B* genotype. The SVR rate according to serum IP-10 alone or *IL28B* genotype alone is indicated on the vertical walls of the graph (white font). SVR rate and number of patients (n SVR / n total subgroup) is shown inside the bar for each subgroup. Likelihood ratio Chi² analysis confirmed response heterogeneity across the sample groups (Chi² = 55; p<0.0001).
**FIGURE 13****.** Graphical summary of a logistic regression analysis fitting sustained virological response (SVR) to peg-interferon and ribavirin treatment based only on serum IP-10 levels (grey curve), or based on a combination level of serum IP-10 (pg/ml) and *IL28B* genotype (CC: red, CT: green, TT: blue). Each patient sample is represented as a circle at the upper (SVR) or lower (non-responder) part of the figure; the colors of the circles represent the *IL28B* genotype. The logistic regression model revealed a significant effect of IP-10 (p<0.0001) and of *IL28B* genotype (p<0.0001), but no significant interaction between IP-10 and *IL28B* genotype (p=0.3).
**FIGURE 14****.** Receiver operating characteristic (ROC) curve analyses for pretreatment serum IP-10, *IL28B* genotype, race, baseline HCV viral load, or a combination of these parameters, in predicting SVR to peg-interferon and ribavirin treatment. The numbers in the legend indicate the ROC area under the curve (AUC) statistic that can be used for model comparison.
**FIGURE 15****.** Predictors of sustained virological response (SVR) to pegIFN and ribavirin therapy. Odds ratios were calculated from a logistic regression model including *IL28B* genotype and baseline (pretreatment) measurements of IP-10, HCV viral load, fibrosis stage (ISHAK), age, gender, alanine transaminase (ALT), steatosis (fat score), and portal and lobular histologic activity index (HAI)

## Claims

1. Method of combining *IL28B* genotype determination with pre-treatment serum level measurement of IP-10 to predict the outcome of sustained virological response (SVR) or non-response to peginterferon and ribavirin for individual patients infected with HCV, wherein the *IL28B* genotype determination comprises the polymorphic marker rs12979860, and wherein Serum IP-10 level within the *IL28B* genotype groups provides additional and independent information regarding the likelihood of SVR, more specifically
• CC carriers with IP-10 < 600 pg/ml have 100% chance for SVR vs. 75% when IP-10 > 600 pg/ml; or wherein
• CT carriers with IP-10 < 600 pg/ml have 79% chance for SVR vs. 34% when IP-10 > 600 pg/ml; or wherein
• TT carriers with IP-10 < 600 pg/ml have 50% chance for SVR vs. 10% when IP-10 > 600 pg/ml.

2. Method according to claim 1 wherein the race parameter is added.

3. Method according to claim 1 wherein the Hepatitis C viral load determination is added.

4. Method according to claim 1 wherein the race parameter and the Hepatitis C viral load determination are added.

5. Method according to claim 4 wherein the combination measurement further discriminates between SVR and non-response to peginterferon and ribavirin for an individual patient infected with HCV, in comparison with any of these two individual markers (*IL28B* genotype, serum IP-10 level).

6. Diagnostic assay for use in the method according to any of the claims 1-5 comprising means for at least the determination or measurement of *IL28B* polymorphism and IP-10 levels in serum from an HCV-infected patient.

## Patentansprüche

1. Verfahren zur Kombination von *IL28B*-Genotyp-Bestimmung mit einer Messung des Serumspiegels von IP-10 vor der Behandlung zur Vorhersage des Ergebnisses einer anhaltendes virologisches Ansprechen (AVA) oder Nicht-Ansprechen auf Peginterferon und Ribavirin für individuelle mit HCV infizierte Patienten, wobei die *IL28B*-Genotyp-Bestimmung den polymorphen Marker rs12979860 umfasst und wobei der IP-10-Serumspiegel innerhalb der *IL28B-*Genotyp-Gruppen zusätzliche und unabhängige Informationen bezüglich der Wahrscheinlichkeit einer AVA liefert, insbesondere
• CC-Träger mit IP-10 < 600 pg/ml eine Chance von 100% auf AVA haben vs. 75% bei IP-10 > 600 pg/ml; oder wobei
• CT-Träger mit IP-10 < 600 pg/ml eine Chance von 79% auf AVA haben vs. 34% bei IP-10 > 600 pg/ml; oder wobei
• TT-Träger mit IP-10 < 600 pg/ml eine Chance von 50% auf AVA haben vs. 10% bei IP-10 > 600 pg/ml.

2. Verfahren nach Anspruch 1, wobei der Rasse-Parameter hinzugefügt wird.

3. Verfahren nach Anspruch 1, wobei die Hepatitis-C-Viruslast-Bestimmung hinzugefügt wird.

4. Verfahren nach Anspruch 1, wobei der Rasse-Parameter und die Hepatitis-C-Viruslast-Bestimmung hinzugefügt werden.

5. Verfahren nach Anspruch 4, wobei die Kombinationsmessung ferner zwischen AVA und Nicht-Ansprechen auf Peginterferon und Ribavirin für einen individuellen mit HCV infizierten Patienten unterscheidet, im Vergleich mit einem dieser beiden Einzelmarker (*IL28B*-Genotyp, IP-10-Serumspiegel).

6. Diagnostischer Test zur Verwendung bei dem Verfahren nach einem der Ansprüche 1-5, umfassend Mittel für wenigstens die Bestimmung oder Messung von *IL28B*-Polymorphismus und IP-10-Spiegeln im Serum von einem HCV-infizierten Patienten.

## Revendications

1. Procédé de combinaison de la détermination du génotype *IL28B* avec la mesure du taux sérique de IP-10 pré-traitement pour prédire le résultat de réponse virologique soutenue (RVS) ou non-réponse au PEG-interféron et à la ribavirine pour des patients individuels infectés par VHC, dans lequel la détermination du génotype *IL28B* comprend le marqueur polymorphe rs12979860, et dans lequel le taux sérique de IP-10 dans les groupes de génotype *IL28B* fournit des informations additionnelles et indépendantes concernant la probabilité de RVS, plus spécifiquement
• les porteurs CC avec IP-10 < 600 pg/ml ont 100 % de probabilité de RVS vs. 75 % lorsque IP-10 > 600 pg/ml ; ou dans lequel
• les porteurs CT avec IP-10 < 600 pg/ml ont 79 % de probabilité de RVS vs. 34 % lorsque IP-10 > 600 pg/ml ; ou dans lequel
• les porteurs TT avec IP-10 < 600 pg/ml ont 50 % de probabilité de RVS vs. 10 % lorsque IP-10 > 600 pg/ml.

2. Procédé selon la revendication 1 dans lequel le paramètre de race est ajouté.

3. Procédé selon la revendication 1 dans lequel la détermination de charge virale de l'hépatite C est ajoutée.

4. Procédé selon la revendication 1 dans lequel le paramètre de race et la détermination de charge virale de l'hépatite C sont ajoutés.

5. Procédé selon la revendication 4 dans lequel la mesure de combinaison distingue en outre RVS et la non-réponse au PEG-interféron et à la ribavirine pour un patient individuel infecté par VHC, en comparaison avec l'un quelconque de ces deux marqueurs individuels (génotype *IL28B,* taux sérique de IP-10).

6. Dosage diagnostique pour utilisation dans le procédé selon l'une quelconque des revendications 1 à 5 comprenant des moyens pour au moins la détermination ou la mesure du polymorphisme de *IL28B* et des taux de IP-10 dans le sérum chez un patient infecté par VHC.
